# EUROPEAN PATENT APPLICATION

(11) **EP 4 740 831 A1**
(43) Date of publication of application: **13.05.2026**
(21) Application number: 23944395.5
(22) Date of filing: 06.07.2023
(51) Int. Cl.: A61B 1/045

(54) **DIAGNOSTIC IMAGING ASSISTANCE DEVICE, DIAGNOSTIC IMAGING ASSISTANCE SYSTEM, AND DIAGNOSTIC IMAGING ASSISTANCE METHOD**

(71) Applicant: Olympus Medical Systems Corp., Tokyo 192-8507 (JP)
(72) Inventor: SUZUKI, Hiroshi, Hachioji-shi, Tokyo 192-8507 (JP); MATSUSHITA, Akira, Hachioji-shi, Tokyo 192-8507 (JP); MIYAZAWA, Shingo, Hachioji-shi, Tokyo 192-8507 (JP); YAJI, Tsuyoshi, Hachioji-shi, Tokyo 192-8507 (JP)
(74) Representative: Gunzelmann, Rainer
(86) International application number: PCT/JP2023/025097
(87) International publication number: WO 2025/009147

(57) **Abstract**

An image diagnosis assistance apparatus 5 includes: a captured image acquisition unit 514 configured to acquire a captured image captured by an imaging device 2 configured to image a subject; a movement determination unit 510 configured to determine magnitude of relative movement between the imaging device 2 and the subject; an image processing unit 515 configured to execute image processing on the captured image to output a processed image; an image generation unit 516 configured to generate an image for diagnosis based on the processed image; and an estimation unit 517 configured to estimate a diagnostic candidate area serving as a diagnostic candidate in the processed image by executing an estimation process for the processed image using a trained model. An input frame rate for the processed image on which high-definition processing is sequentially executed at the image generation unit 516 and an input frame rate for the processed image input sequentially to the estimation unit 517 differ from each other according to the magnitude of the movement.

## Description

### Field

The present invention relates to an image diagnosis assistance apparatus, an image diagnosis assistance system, and an image diagnosis assistance method. Background

Image recognition techniques based on artificial intelligence (AI) have been proposed recently in the medical field (see, for example, Patent Literature 1).

In a technique described in Patent Literature 1, an estimation process using a trained model for a capture image captured by an endoscope is executed, and a diagnostic candidate area of, for example, a lesion in the captured image is thereby estimated.

### Citation List

### Patent Literature

Patent Literature 1: International Publication Pamphlet No. WO 2022/004056

### Summary

### Technical Problem

In a case where an image recognition technique based on AI is utilized, for an observation area of low importance, a medical doctor may increase the observation speed and withdraw the endoscope by relying on the image recognition technique based on AI. However, in a case where the endoscope is withdrawn rapidly, precision of estimation of a diagnostic candidate area of, for example, a lesion by the image recognition technique based on AI is reduced by, for example, image blur due to the rapid movement. Therefore, the endoscope is unable to be withdrawn rapidly from the observation area of low importance and the burden on the medical doctor is unable to be reduced.

The present invention has been made in view of the above, and an object thereof is to provide an image diagnosis assistance apparatus, an image diagnosis assistance system, and an image diagnosis assistance method that enable assistance for diagnosis, the assistance reducing burdens on medical doctors.

### Solution to Problem

To solve the problem described above and to achieve the object, an image diagnosis assistance apparatus according to the present invention includes: a captured image acquisition unit configured to acquire a captured image captured by an imaging device configured to image a subject; a movement determination unit configured to determine magnitude of relative movement between the imaging device and the subject; an image processing unit configured to execute image processing on the captured image to output a processed image; an image generation unit configured to generate, based on the processed image, an image for diagnosis; and an estimation unit configured to estimate a diagnostic candidate area serving as a diagnostic candidate in the processed image, by executing an estimation process for the processed image using a trained model. An input frame rate for the processed image with which the image generation unit sequentially executes a process of generating the image for diagnosis, and an input frame rate for the processed image sequentially input to the estimation unit differ from each other according to the magnitude of the movement.

An image diagnosis assistance apparatus according to the present invention includes: a captured image acquisition unit configured to acquire a captured image captured by an imaging device configured to image a subject; a movement determination unit configured to determine magnitude of relative movement between the imaging device and the subject; an image processing unit configured to execute image processing on the captured image to output a processed image; an image generation unit configured to generate a high-definition image higher in definition than the processed image by executing high-definition processing on the processed image; an estimation unit configured to estimate a diagnostic candidate area serving as a diagnostic candidate in the processed image by executing an estimation process for the processed image using a trained model; and a display control unit configured to generate a display image based on the high-definition image and the diagnostic candidate area. The display control unit is configured to change a form of the display image according to the magnitude of the movement.

An image diagnosis assistance system according to the present invention includes: an imaging device configured to generate a captured image by imaging a subject; and an image diagnosis assistance apparatus configured to process the captured image. The image diagnosis assistance apparatus includes: a captured image acquisition unit configured to acquire the captured image; a movement determination unit configured to determine magnitude of relative movement between the imaging device and the subject; an image processing unit configured to execute image processing on the captured image to output a processed image; an image generation unit configured to generate a high-definition image higher in definition than the processed image by executing high-definition processing on the processed image; and an estimation unit configured to estimate a diagnostic candidate area serving as a diagnostic candidate in the processed image by executing an estimation process for the processed image using a trained model, and an input frame rate for the processed image on which the image generation unit sequentially executes the high-definition processing and an input frame rate for the processed image sequentially input to the estimation unit differ from each other according to the magnitude of the movement.

An image diagnosis assistance method according to the present invention is an image diagnosis assistance method executed by an image diagnosis assistance apparatus. The image diagnosis assistance method includes: a step of acquiring a captured image captured by an imaging device configured to image a subject; a step of determining magnitude of relative movement between the imaging device and the subject; a step of executing image processing on the captured image to output a processed image; a step of generating a high-definition image higher in definition than the processed image by executing high-definition processing on the processed image; and a step of estimating a diagnostic candidate area serving as a diagnostic candidate in the processed image by executing an estimation process for the processed image using a trained model. An input frame rate for the processed image on which the high-definition processing is sequentially executed at an image generation unit configured to execute the high-definition processing and an input frame rate for the processed image sequentially input to an estimation unit configured to execute the estimation process differ from each other according to the magnitude of the movement.

### Advantageous Effects of Invention

An image diagnosis assistance apparatus, an image diagnosis assistance system, and an image diagnosis assistance method, according to the present invention enable assistance for diagnosis, the assistance reducing burdens on medical doctors.

### Brief Description of Drawings

FIG. 1 is a diagram illustrating a configuration of an endoscope system according to an embodiment.
FIG. 2 is a diagram illustrating a configuration of the endoscope system according to the embodiment.
FIG. 3 is a flowchart illustrating an image diagnosis assistance method.
FIG. 4 is a diagram illustrating the image diagnosis assistance method.
FIG. 5 is a diagram illustrating the image diagnosis assistance method.
FIG. 6 is a diagram illustrating a specific example of a display image.
FIG. 7 is a diagram illustrating a specific example of display images.
FIG. 8 is a diagram illustrating a specific example of display images.
FIG. 9 is a diagram illustrating a specific example of a display image.
FIG. 10 is a diagram illustrating a first modified example of the embodiment.
FIG. 11 is a diagram illustrating the first modified example of the embodiment.
FIG. 12 is a diagram illustrating a second modified example of the embodiment.

### Description of Embodiments

Embodiments of the present invention (hereinafter, embodiments) will be described hereinafter with reference to the drawings. The present invention is not to be limited by the embodiments described hereinafter. Like reference signs will be assigned to like parts in the drawings.

### Configuration of Endoscope System

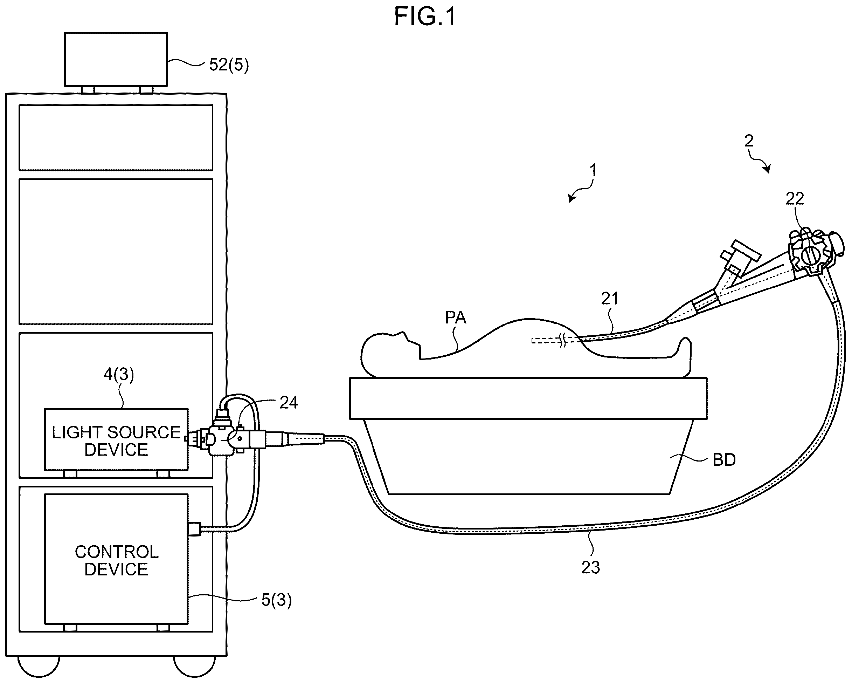

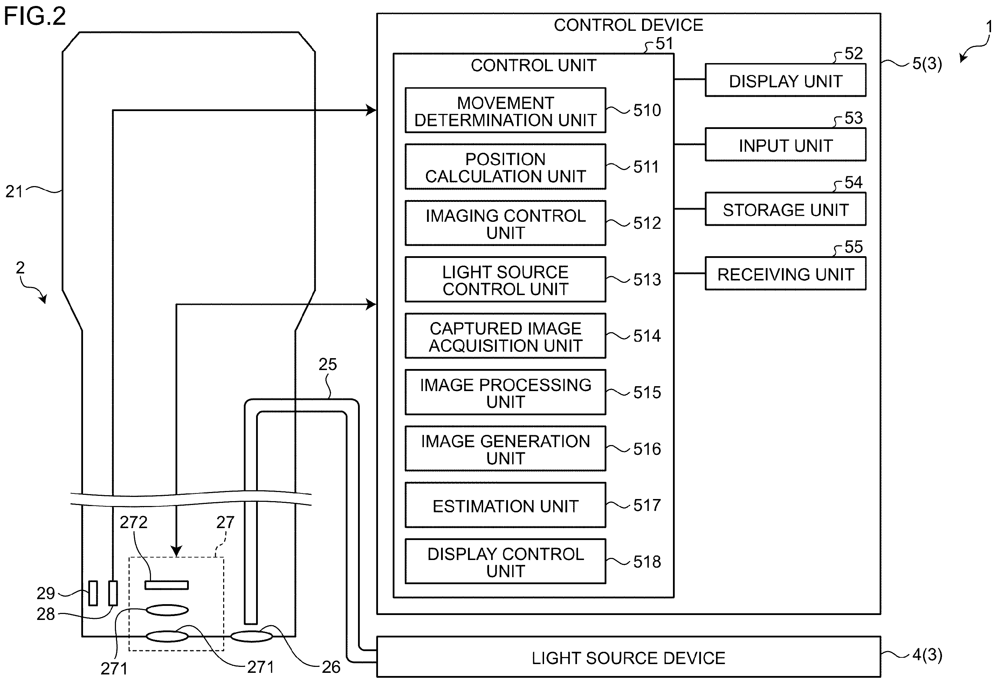

FIG. 1 and FIG. 2 are diagrams illustrating a configuration of an endoscope system 1 according to an embodiment.

The endoscope system 1 corresponds to an image diagnosis assistance system according to the present invention. The endoscope system 1 is a system that is used in the medical field and is for observation of the inside (the large intestine in this embodiment) of the body of a subject PA (FIG. 1) who is a patient on a bed BD (FIG. 1). The endoscope system 1 includes, as illustrated in FIG. 1 and FIG. 2, an endoscope 2 and a processing device 3.

The endoscope 2 corresponds to an imaging device according to the present invention. In this embodiment, the endoscope 2 is a so-called flexible endoscope. Part of the endoscope 2 is inserted in the body of the subject PA, and the endoscope 2 images the inside of the body and outputs an image signal generated by this imaging. The endoscope 2 includes, as illustrated in FIG. 1 and FIG. 2, an insertion unit 21, an operating unit 22, a universal cord 23, and a connector unit 24. Illustration of the operating unit 22, the universal cord 23, and the connector unit 24 has been omitted in FIG. 2 for convenience of description.

At least part of the insertion unit 21 has flexibility and the insertion unit 21 is a portion to be inserted in the body of the subject PA. A light guide 25, an illumination lens 26, an imaging unit 27, and first and second sensors 28 and 29 have been provided in the insertion unit 21, as illustrated in FIG. 2.

The light guide 25 is laid from the insertion unit 21 to the connector unit 24 through the operating unit 22 and the universal cord 23. On end of the light guide 25 is positioned in a distal end portion of the insertion unit 21. Furthermore, in a state where the endoscope 2 has been connected to the processing device 3, the other end of the light guide 25 is positioned in the processing device 3. The light guide 25 transmits observation light supplied from a light source device 4 in the processing device 3 from the other end to the one end of the light guide 25.

The illumination lens 26 is opposed to the one end of the light guide 25 in the insertion unit 21. The illumination lens 26 outputs the observation light transmitted through the light guide 25 to the inside of the body of the subject PA.

The imaging unit 27 is provided in the distal end portion of the insertion unit 21. The imaging unit 27 images the inside of the body of the subject PA and outputs an image signal generated by this imaging. The imaging unit 27 includes, as illustrated in FIG. 2, a lens unit 271 and an imaging element 272.

The lens unit 271 takes in returned light (a subject image) of the observation light output to the inside of the body of the subject PA from the illumination lens 26, and forms the subject image on a light receiving surface of the imaging element 272.

The imaging element 272 includes, for example, a charge coupled device (CCD) or a complementary metal oxide semiconductor (CMOS) that receives the subject image and converts the subject image into an electric signal, and the imaging element 272 generates an image signal by capturing the subject image. An image signal generated by the imaging unit 27 will hereinafter be referred to as a captured image.

The first sensor 28 is a sensor used in determination of magnitude of relative movement between the insertion unit 21 and a subject. In this embodiment, the first sensor 28 is provided at a distal end of the insertion unit 21 and includes an acceleration sensor or an angular velocity sensor.

The second sensor 29 is a sensor used in calculation of a distal end position of the insertion unit 21. In this embodiment, the second sensor 29 includes a magnetic coil that generates magnetism.

The operating unit 22 is connected to a proximal end portion of the insertion unit 21. The operating unit 22 receives various operations for the endoscope 2.

The universal cord 23 is a cord that extends from the operating unit 22, in a direction different from a direction in which the insertion unit 21 extends, and the cord has, provided therein, for example, the light guide 25 and a signal line electrically connecting the imaging unit 27 and a control device 5 in the processing device 3.

The connector unit 24 is provided at an end portion of the universal cord 23 and is detachably connected to the processing device 3.

The processing device 3 includes, as illustrated in FIG. 2, the light source device 4, and the control device 5.

Under control by the control device 5, the light source device 4 supplies observation light to be emitted to a subject, to the other end of the light guide 25. Examples of the observation light may include white light, excitation light to excite a fluorescent agent, such as indocyanine green, and narrowband light used in narrowband imaging (NBI).

The control device 5 corresponds to an image diagnosis assistance apparatus according to the present invention. The control device 5 includes, as illustrated in FIG. 2, a control unit 51, a display unit 52, an input unit 53, a storage unit 54, and a receiving unit 55.

The control unit 51 is configured to include a controller, such as a central processing unit (CPU) or a microprocessing unit (MPU), or an integrated circuit, such as an application specific integrated circuit (ASIC) or a field programmable gate array (FPGA), and controls the overall operation of the endoscope system 1. The control unit 51 includes, as illustrated in FIG. 2, a movement determination unit 510, a position calculation unit 511, an imaging control unit 512, a light source control unit 513, a captured image acquisition unit 514, an image processing unit 515, an image generation unit 516, an estimation unit 517, and a display control unit 518. Functions of the movement determination unit 510, the position calculation unit 511, the imaging control unit 512, the light source control unit 513, the captured image acquisition unit 514, the image processing unit 515, the image generation unit 516, the estimation unit 517, and the display control unit 518, which are in the control unit 51, will be described in "Image Diagnosis Assistance Method", and "Specific Examples of Display Images", described later.

The display unit 52 corresponds to a notification unit according to the present invention. The display unit 52 is, for example, a liquid crystal display (LCD) or an electroluminescence (EL) display, and displays, under control by the control unit 51, a display image generated by the control unit 51.

The input unit 53 corresponds to an operation reception unit according to the present invention. The input unit 53 is configured using a keyboard, a mouse, a switch, and/or a touch panel, for example, and receives a user operation from a user, such as an operating surgeon. The input unit 53 outputs an operation signal corresponding to the user operation, to the control unit 51.

The storage unit 54 stores various programs executed by the control unit 51 and information needed in processing by the control unit 51, for example.

The receiving unit 55 is a receiving unit used with the second sensor 29 in calculation of a distal end position of the insertion unit 21 and receives, under control by the control unit 51, magnetism transmitted from the second sensor 29.

### Image Diagnosis Assistance Method

An image diagnosis assistance method executed by the control device 5 described above will be described next.

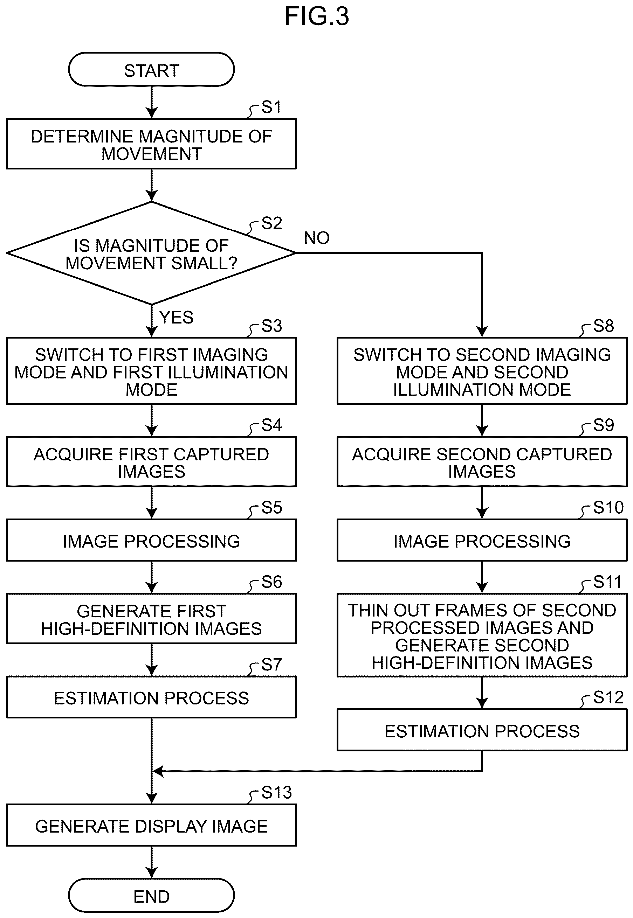

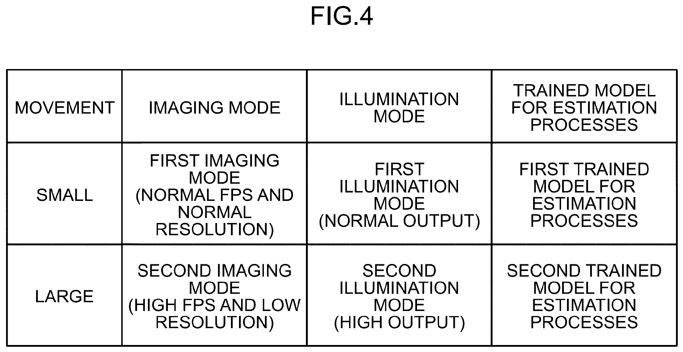

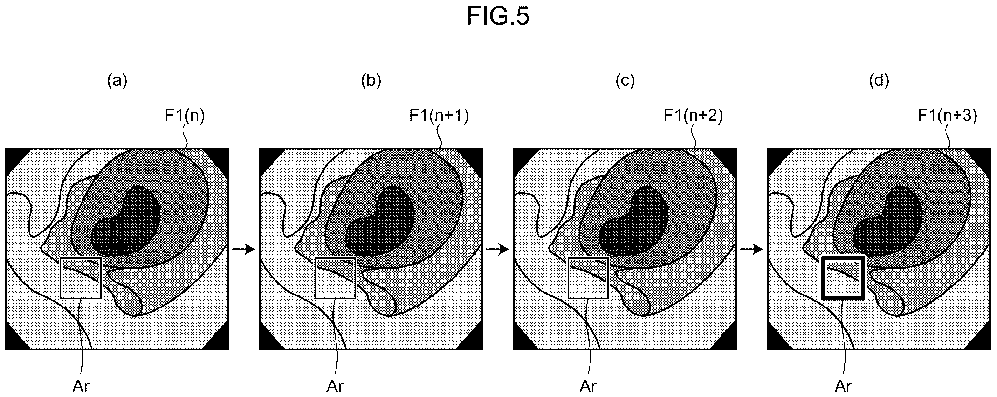

FIG. 3 is a flowchart illustrating the image diagnosis assistance method. FIG. 4 and FIG. 5 are diagrams illustrating the image diagnosis assistance method.

Firstly, the movement determination unit 510 determines magnitude of relative movement between the insertion unit 21 and a subject on the basis of a signal output from the first sensor 28 (Step S1).

In a case where the magnitude of the movement determined at Step S1 is less than a predetermined threshold (in a case where the magnitude of the movement is small) (Step S2: Yes), the imaging control unit 512 and the light source control unit 513 switches an imaging mode to a first imaging mode and switches an illumination mode to a first illumination mode (Step S3).

For the determination of the magnitude of the movement at Step S1, the magnitude of the movement is determined at the time of a withdrawal operation. At the time of an insertion operation, a posture change in an image is large because insertion is performed while a biologically gentle path is searched for. On the contrary, at the time of the withdrawal operation, a posture change in an image is small because withdrawal is able to be performed by a comparatively simple operation and observation and diagnosis thus become comparatively easy. The magnitude of the movement may be determined regardless of the direction of the operation described above.

The first imaging mode is a mode where resolution is resolution (normal resolution) at which all of pixels in an effective pixel area are used and a frame rate (FPS) of imaging by the imaging unit 27 is a normal frame rate (normal FPS), such as 60 (FPS), as illustrated in FIG. 4. That is, at Step S3, the imaging control unit 512 controls operation of the imaging unit 27 to switch the imaging mode to the first imaging mode.

Furthermore, as illustrated in FIG. 4, the first illumination mode is a mode where output of observation light from the light source device 4 is normal output. That is, at Step S3, the light source control unit 513 controls operation of the light source device 4 to switch the illumination mode to the first illumination mode.

After Step S3, the captured image acquisition unit 514 sequentially acquires captured images generated by the imaging unit 27 imaging returned light of observation light from the inside of the body of the subject PA in a state where the observation light of the normal output is emitted to the inside of the body of the subject PA from the light source device 4 (Step S4). The captured images are images of normal resolution captured at the normal FPS because the imaging mode is in the first imaging mode. These captured images will hereinafter be referred to as first captured images for convenience of description.

After Step S4, the image processing unit 515 executes image processing on the first captured images sequentially acquired at Step S4 (Step S5). The first captured images that have been subjected to the image processing by the image processing unit 515 at Step S5 will hereinafter be referred to as first processed images. The image processing unit 515 then outputs the first processed images to each of the image generation unit 516 and the estimation unit 517.

The image processing may be, for example, known image processing, such as gain adjustment, white balance adjustment, gamma correction, contour enhancement correction, or enlargement and reduction adjustment.

After Step S5, the image generation unit 516 generates images suitable for display from the first processed images and outputs the images (Step S6). In this embodiment, by using a trained model for high-definition processing, at Step S6, the image generation unit 516 estimates image quality of the first processed images input, and in a case where the image quality of the first processed images is estimated to be of low definition, by executing high-definition processing, the image generation unit 516 generates high-definition images increased in image quality by executing high-definition processing as if the high-definition images were generated by an endoscope (hereinafter, referred to as a high-quality endoscope) that generates high-quality captured images. On the contrary, in a case where the image quality of the first processed images input is estimated to be of high definition, the image generation unit 516 outputs images having the image quality of the first processed images without executing the high-definition processing. The high-definition images generated at Step S6 will hereinafter be referred to as first high-definition images. That is, the first high-definition images and the above mentioned images having the image quality of the first processed images correspond to images for diagnosis according to the present invention. Furthermore, the high-definition processing corresponds to a process of generating an image for diagnosis, according to the present invention. The first high-definition images are images captured at the normal FPS because the imaging mode is in the first imaging mode.

The trained model for high-definition processing is stored in the storage unit 54 beforehand. Specifically, the trained model for high-definition processing is a learning model that has been trained by a training process repeatedly executed for the learning model using multiple pairs of training images and sets of training data, each of the multiple pairs being a pair of a training image and a set of training data. The training images are images obtained by reducing image quality of captured images (hereinafter, referred to as high-quality images) generated by high-quality endoscopes to the image quality corresponding to the first processed images. The sets of training data are those high-quality images. The learning model used in the training process is, for example, a convolutional neural network (CNN). The trained model for high-quality processing includes a weight file (training parameters) having weight values and bias values of each layer of the CNN.

The neural network used in the training process for generating the trained model for high-quality processing is not necessarily a CNN and any other neural network may be adopted instead. Furthermore, any of various publicly known learning algorithms may be adopted as a machine learning algorithm in the neural network. For example, a supervised learning algorithm using the backpropagation method may be adopted.

Furthermore, after Step S5, by executing an estimation process for the first processed images using a first trained model for estimation processes, the estimation unit 517 estimates a diagnostic candidate area serving as a diagnostic candidate for each predetermined area in the first processed images (Step S7).

FIG. 3 illustrates that Step S7 is executed after Step S6, but in practice, Steps S6 and S7 are executed substantially simultaneously in parallel.

The first trained model for estimation processes corresponds to a trained model according to the present invention. The first trained model for estimation processes is stored in the storage unit 54 beforehand. Specifically, the first trained model for estimation processes is a learning model that has been trained by a training process repeatedly executed for the learning model using multiple pairs of training images and sets of training data, each of the multiple pairs being a pair of a training image and a set of training data. The training image is a captured image obtained by imaging of the inside of a living body. The set of training data is a set of data including annotations of a classification class, a ground-truth position, and a size of, for example, a lesion in the training image. The learning model used in the training process is, for example, a CNN. The trained model for estimation processes includes a weight file (training parameters) having weight values and bias values of each layer of the CNN.

The neural network used in the training process for generating the first trained model for estimation processes is not necessarily a CNN and any other neural network may be adopted instead. For example, a neural network, such as a deep neural network (DNN), Transformer, or a generative adversarial network (GAN), may be adopted as appropriate. Furthermore, any of various publicly known learning algorithms may be adopted as a machine learning algorithm in the neural network. For example, a supervised learning algorithm using the backpropagation method may be adopted.

Furthermore, by executing an estimation process, the estimation unit 517 outputs reliability of a diagnostic candidate area for each predetermined area in the first processed images.

The reliability of a diagnostic candidate area is a value indicating the level of reliability.

Specifically, the reliability is a value indicating accuracy of image recognition in the diagnostic candidate area and may also be said to be an index representing a value indicating predicted probability that an object serving as an image belongs to a specific class. Whether the object has been accurately recognized in the area is able to be determined from the reliability of the diagnostic candidate area.

At Step S7, the estimation unit 517 then estimates a diagnostic candidate area that is an area where reliability is consecutively at a reliability threshold or more for a first number of frames, among the reliability of each predetermined area in the first processed images output by the estimation process. In a case where there is instantaneously an area with reliability at the reliability threshold or more for just one frame in a time-series, there is a possibility that the area estimated is an area that has been falsely detected, and this area estimated is thus not treated as the diagnostic candidate area.

FIG. 5 illustrates an example of a case where the first number of frames is "4". That is, even in a case where reliability of an area Ar in a first processed image F1(n) of an n-th frame is at the reliability threshold or more, the estimation unit 517 does not estimate the area Ar as a diagnostic candidate area yet. In a case where the reliability of the area Ar is consecutively at the reliability threshold or more for four frames from the first processed image F1(n) of the n-th frame to a first processed image F1(n+3) of an (n+3)-th frame, the estimation unit 517 then estimates the area Ar as a diagnostic candidate area.

As described above, in the case where the magnitude of the movement is small, an input frame rate for the first processed images on which high-definition processing is executed sequentially at the image generation unit 516 and an input frame rate for the first processed images sequentially input to the estimation unit 517 are the same at the normal FPS, such as 60 (FPS).

On the contrary, in a case where the magnitude of the movement determined at Step S1 is at the predetermined threshold or more (in a case where the magnitude of the movement is large) (Step S2: No), the imaging control unit 512 and the light source control unit 513 switches the imaging mode to the second imaging mode and the illumination mode to the second illumination mode (Step S8).

The second imaging mode is, as illustrated in FIG. 4, a mode where the imaging frame rate (FPS) of the imaging unit 27 is at an imaging frame rate (high FPS), such as 120, 240, or 480 (FPS), which is larger than the normal FPS. Furthermore, in the second imaging mode, to maintain readability even at the high FPS, the resolution is maintained at resolution (low resolution) with the number of pixels less than that of the normal resolution by decimated readout or pixel summation. That is, at Step S8, the imaging control unit 512 controls operation of the imaging unit 27 to switch the imaging mode to the second imaging mode.

Furthermore, as illustrated in FIG. 4, the second illumination mode is a mode where observation light from the light source device 4 is pulsed light higher in output than the normal output. That is, at Step S8, the light source control unit 513 controls operation of the light source device 4 to switch the illumination mode to the second illumination mode.

After Step S8, the captured image acquisition unit 514 sequentially acquires captured images generated by the imaging unit 27 imaging returned light of observation light from the inside of the body of the subject PA in a state where the observation light is emitted to the inside of the body of the subject PA from the light source device 4, the observation light being the pulsed light high in output (Step S9). The captured images are images low in resolution captured at the high FPS because the imaging mode is in the second imaging mode. These captured images will hereinafter be referred to as second captured images, for convenience of description.

After Step S9, the image processing unit 515 executes image processing sequentially on the second captured images acquired sequentially at Step S9, similarly to Step S5 (Step S10). The second captured images that have been subjected to the image processing by the image processing unit 515 at Step S10 will hereinafter be referred to as second processed images. The image processing unit 515 then outputs the second processed images to each of the image generation unit 516 and the estimation unit 517.

After Step S10, the image generation unit 516 sequentially reduces the input frame rate at which high-definition processing is executed by thinning out frames of the second processed images input, in order to achieve a frame rate enabling display at the display unit 52. Furthermore, similarly to Step S6, by using the trained model for high-definition processing, the image generation unit 516 estimates image quality of the second processed images input, and in a case where image quality of the second processed images has been estimated to be of low definition, the image generation unit 516 generates high-definition images that have been increased in image quality (increased in resolution) by executing high-definition processing, as if the high-definition images were generated by a high-quality endoscope (Step S11). On the contrary, in a case where the image quality of the second processed images input is estimated to be of high definition, the image generation unit 516 outputs images having the image quality of the second processed images without executing high-definition processing. As described above, because the images are low in resolution in the second imaging mode, the second processed images will often be subjected to high-definition processing executed by the image generation unit 516. At Steps S6 and S11, execution of high-definition processing is determined according to a result of estimation of whether or not the first or second processed images have image quality of high definition, but the embodiment is not to be limited to this configuration. For example, whether or not high-definition processing is to be executed may be changed according to the first imaging mode or the second imaging mode. The high-definition images generated at Step S11 will hereinafter be referred to as second high-definition images. That is, the second high-definition images and the above mentioned images having the image quality of the second processed images correspond to images for diagnosis according to the present invention. Because the imaging mode is the second imaging mode and frames have been thinned out by the image generation unit 516, the input frame rate for the second high-definition images according to the embodiment is at the normal FPS, such as 60 (FPS).

Furthermore, after Step S10, by executing an estimation process for the second processed images using a second trained model for estimation processes, the estimation unit 517 estimates a diagnostic candidate area serving as a diagnostic candidate for each predetermined area in the second processed images (Step S12).

FIG. 3 illustrates that Step S12 is executed after Step S11, but in practice, Steps S11 and S12 are executed substantially simultaneously in parallel.

The second trained model for estimation processes corresponds to a trained model according to the present invention. The second trained model for estimation processes is stored in the storage unit 54 beforehand. The second trained model for estimation processes is a model whose layers each output a feature map having a size different from that of the first train model for estimation processes because the resolution of the second processed images is lower than the resolution of the first processed images. Therefore, the second trained model for estimation processes is a model having a layer structure and the number of channels that are different from those of the first trained model for estimation processes, the layer structure and the number of channels being of its network model of its neural network, even though the second trained model for estimation processes is a trained model generated using training images and sets of training data similar to those of the first trained model for estimation processes. That is, the estimation unit 517 changes the trained model for estimation processes according to the magnitude of the movement determined at Step S1 (FIG. 4).

Furthermore, at Step S12, the estimation unit 517 estimates a diagnostic candidate area that is an area where reliability is consecutively at a reliability threshold or more for a second number of frames smaller than a first number of frames, among the reliability of each predetermined area in the second processed images output by the estimation process. That is, the estimation unit 517 switches the number of frames to the first number of frames or the second number of frames, according to the magnitude of the movement determined at Step S1.

As described above, in the case where the magnitude of the movement is large, an input frame rate for the second processed images on which high-definition processing is executed sequentially at the image generation unit 516 and an input frame rate for the second processed images sequentially input to the estimation unit 517 are different from each other. Specifically, the input frame rate for the second processed images on which high-definition processing is executed sequentially at the image generation unit 516 is smaller than the input frame rate for the second processed images sequentially input to the estimation unit 517.

After Steps S6 and S7, or after Steps S11 and S12, the display control unit 518 generates a display image to be displayed by the display unit 52 (Step S13).

Specifically, after Steps S6 and S7, the display control unit 518 generates the display image on the basis of a first high-definition image generated at Step S6 and a diagnostic candidate area estimated at Step S7. Furthermore, after Steps S11 and S12, the display control unit 518 generates a display image on the basis of a second high-definition image generated at Step S11 and a diagnostic candidate area estimated at Step S12.

Details of the display image will be described in "Specific Examples of Display Images" described later.

### Specific Examples of Display Images

Specific examples of display images displayed on the display unit 52 will be described next.

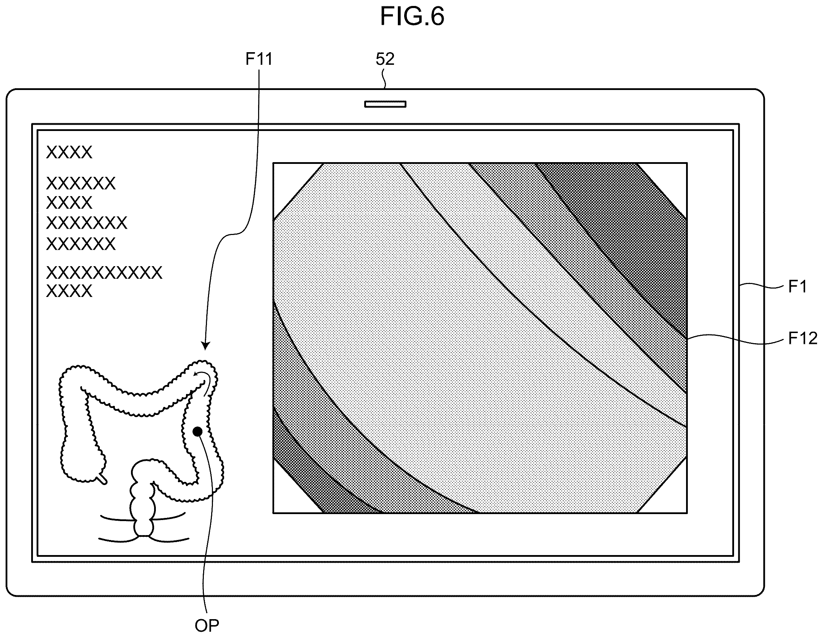

FIG. 6 to FIG. 9 are diagrams illustrating specific examples of display images.

For example, the display control unit 518 generates a display image F1 illustrated in FIG. 6 in the image diagnosis assistance method described above. The display control unit 518 then causes the display unit 52 to display the display image F1.

The display image F1 includes an observation position image F11 and a diagnostic image F12, as illustrated in FIG. 6.

The observation position image F11 is an image having a current observation position (a distal end position of the insertion unit 21) OP superposed on an image depicting a shape of an observation target (the large intestine in the case for this embodiment). Specifically, the position calculation unit 511 calculates the distal end position OP of the insertion unit 21 by a publicly known technique, on the basis of magnetism transmitted from the second sensor 29 and received at the receiving unit 55. The display control unit 518 then generates the observation position image F11 having the distal end position OP (the current observation position) of the insertion unit 21 calculated at the position calculation unit 511, the distal end position OP having been superposed on the image depicting the shape of the observation target whose position has been determined beforehand.

The diagnostic image F12 is differently formed according to the magnitude of the movement determined at Step S1.

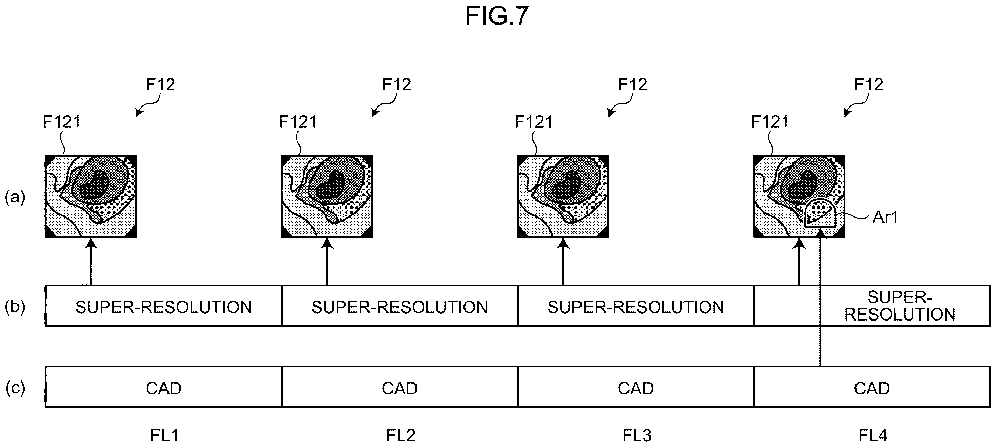

For example, in the case where the magnitude of the movement is small (Step S2: Yes), the diagnostic image F12 will be like images illustrated in FIG. 7. At (a), FIG. 7 illustrates diagnostic images F12 generated sequentially, with its horizontal axis representing time. At (b), FIG. 7 illustrates frames of first high-definition images generated sequentially, with its horizontal axis representing time. For convenience of description, the frames are labelled with text, "super-resolution". In the case where the magnitude of the movement is small, the first processed images are often high in definition as described above and the diagnostic images F12 will have the image quality of the first processed images. At (c), FIG. 7 illustrates frames of first processed images for which an estimation process is executed, with its horizontal axis representing time. For convenience of description, the frames are labelled with text, "CAD".

In the case where the magnitude of the movement is small, as illustrated at (b) and (c) in FIG. 7, the input frame rate for the first processed images on which high-definition processing is sequentially executed at the image generation unit 516 and the input frame rate for the first processed images input sequentially to the estimation unit 517 are the same at the normal FPS, such as 60 (FPS).

In the case where the magnitude of the movement is small, as illustrated in FIG. 7, the display control unit 518 generates a diagnostic image F12 having a diagnostic candidate area Ar1 superposed on a first high-definition image F121, on the basis of the first high-definition image F121 and the diagnostic candidate area Ar1 that result from processing of a first processed image of the same frame (a frame FL4 in the example of FIG. 7) by the image generation unit 516 and the estimation unit 517.

In a case where a diagnostic candidate area Ar1 has been unable to be estimated as a result of an estimation process, the display control unit 518 generates only a first high-definition image F121 as a diagnostic image F12 (frames FL1 to FL3 in the example of FIG. 7).

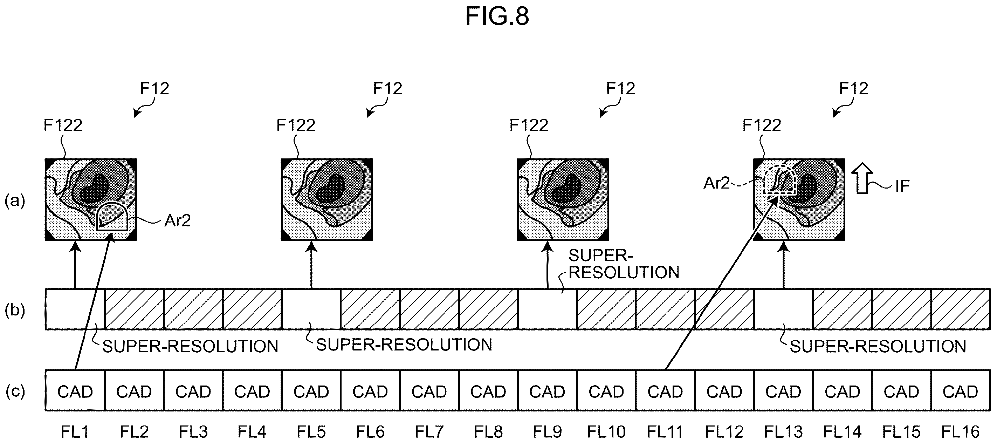

Furthermore, for example, in the case where the magnitude of the movement is large (Step S2: No), the diagnostic image F12 will be like images illustrated in FIG. 8. At (a), FIG. 8 illustrates diagnostic images F12 generated sequentially, with its horizontal axis representing time. At (b), FIG. 8 illustrates frames of second high-definition images generated sequentially, with its horizontal axis representing time. For convenience of description, the frames are labelled with text, "super-resolution". At (c), FIG. 8 illustrates frames of second processed images for which an estimation process is executed, with its horizontal axis representing time. For convenience of description, the frames are labelled with text, "CAD".

In the case where the magnitude of the movement is large, as illustrated at (b) and (c) in FIG. 8, the input frame rate for the second processed images on which high-definition processing is sequentially executed at the image generation unit 516 and the input frame rate for the second processed images input sequentially to the estimation unit 517 are different from each other. In the example of FIG. 8, the input frame rate for the second processed on which high-definition processing is sequentially executed at the image generation unit 516 is 60 (FPS). The input frame rate for the second processed images sequentially input to the estimation unit 517, on the other hand, is 240 (FPS).

In the case where the magnitude of the movement is large, the display control unit 518 generates diagnostic images F12 described hereinafter.

As illustrated in FIG. 8, the display control unit 518 generates a diagnostic image F12 having a diagnostic candidate area Ar2 superposed on a second high-definition image F122, on the basis of the second high-definition image F122 and the diagnostic candidate area Ar2 that result from processing of a second processed image of the same frame (a frame FL1 in the example of FIG. 8) by the image generation unit 516 and the estimation unit 517.

Furthermore, as illustrated in FIG. 8, the display control unit 518 generates a diagnostic image F12 including a second high-definition image F122 and frame position information IF indicating a frame (a frame FL11 in the example of FIG. 8) from which a diagnostic candidate area Ar2 has been estimated, on the basis of the second high-definition image F122 and the diagnostic candidate area Ar2 that result from processing of second processed images of different frames (the frame FL11 and a frame FL13 in the example of FIG. 8) by the image generation unit 516 and the estimation unit 517. In this embodiment, the frame FL11 which is at a time when the insertion unit 21 is withdrawn and from which the diagnostic candidate area Ar2 has been estimated is a frame captured at a site deeper in a second high-definition image F122 than the frame FL13 of the second high-definition image F122. Therefore, information, an arrow indicating the site deeper, is adopted as the frame position information IF. As illustrated by a broken line in FIG. 8, for this diagnostic image F12, the diagnostic candidate area Ar2 may be superposed on the second high-definition image F122 of the frame FL13 which is nearest to the frame FL 11 from which the diagnostic candidate area Ar2 has been estimated.

In a case where a diagnostic candidate area Ar2 is unable to be estimated as a result of an estimation process, the display control unit 518 generates only a second high-definition image F122 as a diagnostic image F12 (frames FL5 to FL9 in the example of FIG. 8).

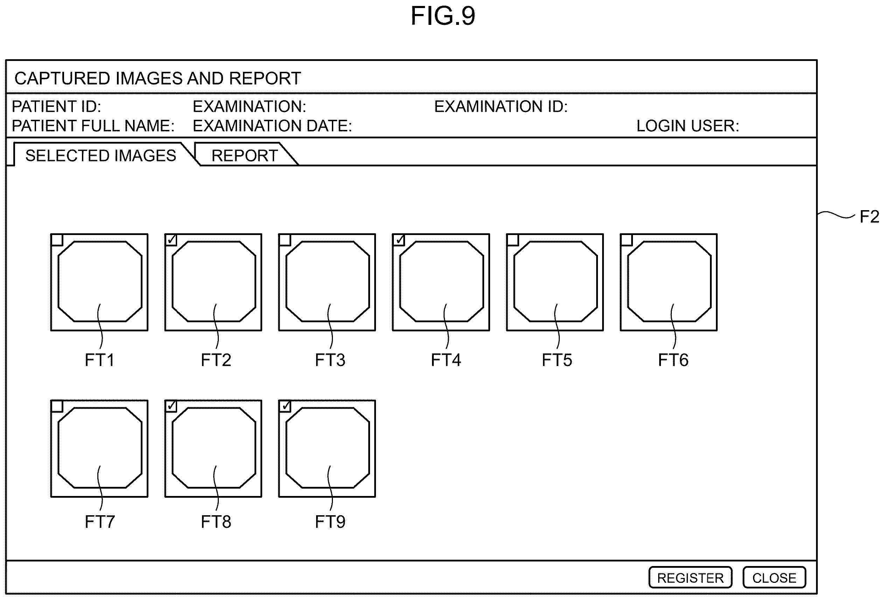

Furthermore, for example, the display control unit 518 generates a display image F2 illustrated in FIG. 9. The display control unit 518 then causes the display unit 52 to display the display image F2.

The control unit 51 stores the diagnostic images F12 into the storage unit 54, the diagnostic images F12 having the diagnostic candidate areas Ar1 and Ar2 superposed on the first high-definition images F121 and the second high-definition images F122.

According to a user operation on the input unit 53, the display control unit 518 generates the display image F2 for display of a list of thumbnail images FT1 to FT9 of the multiple diagnostic images F12 stored in the storage unit 54.

The above described embodiment has the following effects.

In the case where the magnitude of the movement is large, the control device 5 according to the embodiment switches the imaging mode to the second imaging mode and switches the illumination mode to the second illumination mode. Therefore, the control device 5 is able to perform an estimation process for the second processed images without any image blur due to rapid movement of the insertion unit 21 and is thus able precisely estimate any diagnostic candidate area. Furthermore, the control device 5 generates the second high-definition images increased in image quality (increased in resolution) by high-definition processing of the second processed images that have become low in resolution because of the second imaging mode. Therefore, a medical doctor, for example, is able to check the appropriate diagnostic images F12.

Therefore, the control device 5 according to the embodiment enables the insertion unit 21 to be withdrawn promptly from an observation area of low importance and enables diagnostic assistance that reduces burdens on medical doctors.

Furthermore, the control device 5 according to the embodiment changes the trained model for estimation processes according to the magnitude of the movement determined at Step S1.

Therefore, any diagnostic candidate area is able to be appropriately estimated using an appropriate trained model for estimation processes according to the magnitude of the movement.

Furthermore, the control device 5 according to the embodiment switches the number of frames to be used in estimation of a diagnostic candidate area to the first number of frames or the second number of frames, according to the magnitude of the movement determined at Step S1.

Therefore, false estimation of diagnostic candidate areas is able to be minimized.

### Other Embodiments

An embodiment of the present invention has been described thus far, but the present invention should not be limited only to the embodiment described above.

In the above described embodiment, the image diagnosis assistance apparatus according to the present invention is installed in the endoscope system 1 including the insertion unit 21 using a flexible endoscope but the embodiment is not to be limited to this example. For example, the image diagnosis assistance apparatus according to the present invention may be installed in an endoscope system including an insertion unit 21 using a rigid endoscope. Furthermore, the image diagnosis assistance apparatus according to the present invention may be installed in a medical observation system, such as a surgical microscope (see, for example, Japanese Unexamined Patent Application Publication No. 2016-042981) for observation of a predetermined field of view within a subject (within a living body) or on a surface of the subject (a surface of the living body) by magnifying the predetermined field of view.

In the above described embodiment, the image generation unit 516 generates the first and second high-definition images increased in image quality as if they were generated by a high-quality endoscope that generates high quality captured images, by executing high-definition processing on the first and second processed images using the trained model for high-definition processing, but the embodiment is not to be limited to this example.

For example, the image generation unit 516 may generate, as the first and second high-definition images, images that have been subjected to filter processing, such as edge enhancement or image enhancement, contrast-enhanced images, images that have been subjected to filter processing for structural color enhancement, or images that have been subjected to deblurring (deconvolution images).

In the above described embodiment, on the basis of a signal output from the first sensor 28 including an acceleration sensor or an angular velocity sensor, the movement determination unit 510 determines the magnitude of relative movement between the insertion unit 21 and a subject, but the embodiment is not to be limited to this example.

For example, on the basis of captured images, the movement determination unit 510 may determine the magnitude of relative movement between the insertion unit 21 and a subject by a publicly known method, such as the block matching method or the gradient method.

In the above described embodiment, discrimination information enabling discrimination between the time at which the insertion unit 21 is inserted in a body and the time at which the insertion unit 21 is withdrawn from the inside of the body may be included in a diagnostic image F12. Furthermore, in a configuration that may be adopted, in a case where a diagnostic candidate area is estimated at the time of insertion, the distal end position of the insertion unit 21 at the time of the estimation is stored in the storage unit 54 and a notification unit, such as the display unit 52, may perform notification of information indicating that the distal end position of the insertion unit 21 has come closer to the distal end position stored in the storage unit 54 at the time of withdrawal in a case where the distal end position of the insertion unit 21 has come closer to the distal end position stored in the storage unit 54 at the time of withdrawal.

Furthermore, a first or second modified example described hereinafter may be adopted in the above described embodiment.

### First Modified Example

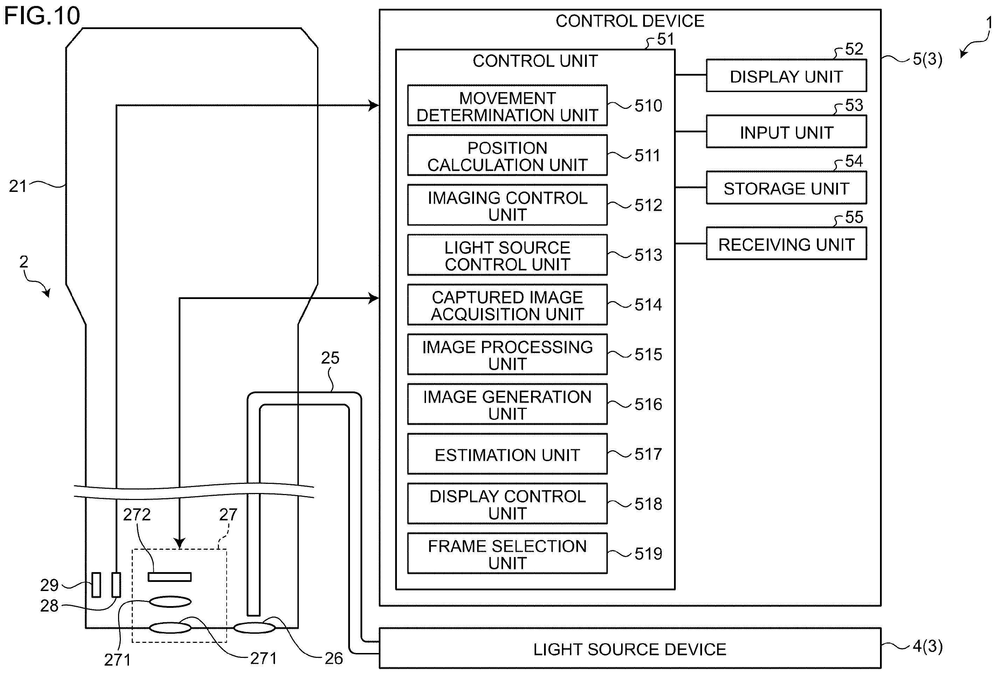

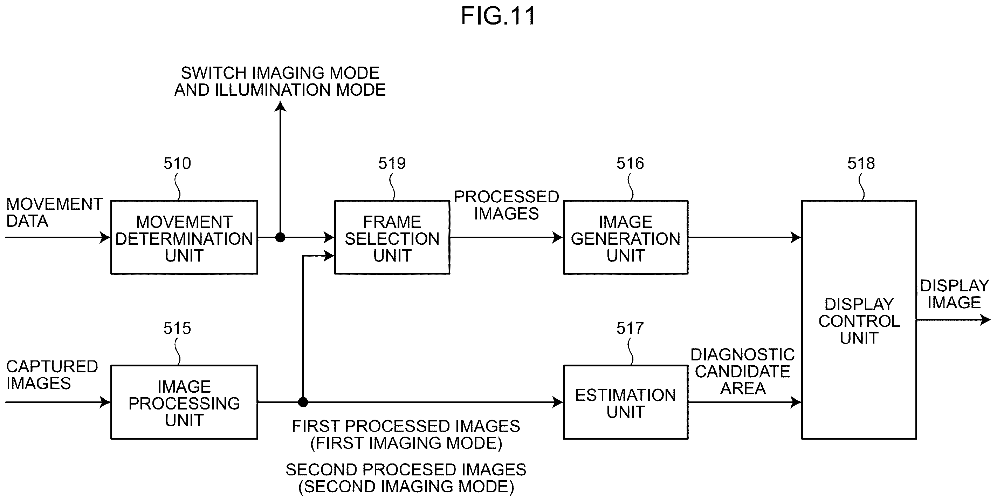

FIG. 10 and FIG. 11 are diagrams illustrating a first modified example of the embodiment. Specifically, FIG. 10 is a diagram corresponding to FIG. 2. FIG. 11 is a block diagram illustrating functions of a control unit 51.

The control unit 51 according to the first modified example has a frame selection unit 519 added to the control unit 51 described above with respect to the embodiment, as illustrated in FIG. 10 and FIG. 11.

In the above described embodiment, in the case where the magnitude of the movement is large, the image generation unit 516 sequentially decreases the input frame rate at which high-definition processing is executed, by thinning out the frames of the second processed images input. That is, the image generation unit 516 itself performs the thinning described above.

In contrast, in this first modified example, the frame selection unit 519, instead of an image generation unit 516, performs the thinning described above. That is, in a case where magnitude of movement is large, the frame selection unit 519 performs the above described thinning and sequentially inputs second processed images at an input frame rate decreased by the thinning, to the image generation unit 516. On the contrary, in a case where the magnitude of the movement is small, the frame selection unit 519 does not perform the thinning described above. That is, with the frame rate being maintained, first processed images are sequentially input to the image generation unit 516.

Furthermore, the image generation unit 516 according to the first modified example estimates image quality of an image input from the frame selection unit 519, in a case where the image quality is estimated to be of low definition, the image generation unit 516 generates and outputs a high-definition image that has been increased in image quality by executing high-definition processing, and in a case where the image quality is estimated to be of high definition, the image generation unit 516 outputs an image having the input image quality without executing high-definition processing. That is, the high-definition image, and the above described image having the input image quality correspond to images for diagnosis, according to the present invention.

In a case where the above described configuration according to the first modified example is adopted, effects similar to those of the embodiment described above are also achieved.

### Second Modified Example

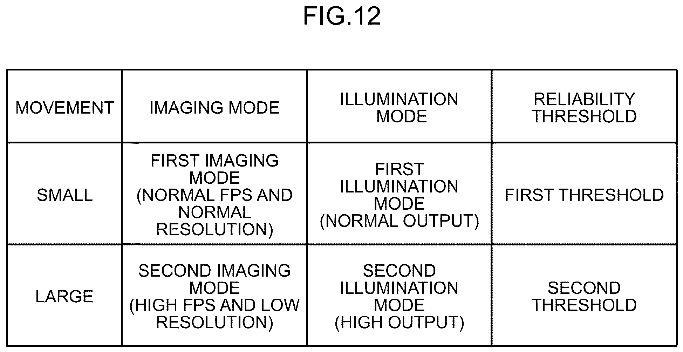

FIG. 12 is a diagram illustrating a second modified example of the embodiment. Specifically, FIG. 12 is a diagram corresponding to FIG. 4.

In the above described embodiment, the estimation unit 517 changes the trained model for estimation processes according to the magnitude of the movement determined at Step S1, but the embodiment is not to be limited to this example.

In the second modified example, an estimation unit 517 uses the same trained model for estimation processes for both a case where magnitude of movement is small and a case where magnitude of movement is large. Furthermore, in the case where the magnitude of the movement is small, the estimation unit 517 uses, as a reliability threshold to be used in an estimation process, a first threshold stored in a storage unit 54. On the contrary, in the case where the magnitude of the movement is large, the estimation unit 517 uses, as the reliability threshold to be used in an estimation process, a second threshold stored in the storage unit 54. The first threshold and the second threshold are thresholds different from each other.

The second modified example described above has, in addition to effects similar to those of the embodiment described above, the following effects.

A control device 5 according to the second modified example changes the reliability threshold to be used in an estimation process, that is, the detection sensitivity for a diagnostic candidate area, according to the magnitude of the movement determined at Step S1.

Therefore, diagnostic candidate areas are able to be appropriately estimated using appropriate reliability thresholds according to the magnitude of the movement.

### Reference Signs List

- 1: ENDOSCOPE SYSTEM
- 2: ENDOSCOPE
- 3: PROCESSING DEVICE
- 4: LIGHT SOURCE DEVICE
- 5: CONTROL DEVICE
- 21: INSERTION UNIT
- 22: OPERATING UNIT
- 23: UNIVERSAL CORD
- 24: CONNECTOR UNIT
- 25: LIGHT GUIDE
- 26: ILLUMINATION LENS
- 27: IMAGING UNIT
- 28: FIRST SENSOR
- 29: SECOND SENSOR
- 51: CONTROL UNIT
- 52: DISPLAY UNIT
- 53: INPUT UNIT
- 54: STORAGE UNIT
- 55: RECEIVING UNIT
- 271: LENS UNIT
- 272: IMAGING ELEMENT
- 510: MOVEMENT DETERMINATION UNIT
- 511: POSITION CALCULATION UNIT
- 512: IMAGING CONTROL UNIT
- 513: LIGHT SOURCE CONTROL UNIT
- 514: CAPTURED IMAGE ACQUISITION UNIT
- 515: IMAGE PROCESSING UNIT
- 516: IMAGE GENERATION UNIT
- 517: ESTIMATION UNIT
- 518: DISPLAY CONTROL UNIT
- 519: FRAME SELECTION UNIT
- Ar, Ar1, Ar2: DIAGNOSTIC CANDIDATE AREA
- BD: BED
- F1, F2: DISPLAY IMAGE
- F11: OBSERVATION POSITION IMAGE
- F12: DIAGNOSTIC IMAGE
- F121: FIRST HIGH-DEFINITION IMAGE
- F122: SECOND HIGH-DEFINITION IMAGE
- FT1 TO FT9: THUMBNAIL IMAGES
- IF: FRAME POSITION INFORMATION
- OP: OBSERVATION POSITION
- PA: SUBJECT

## Claims

1. An image diagnosis assistance apparatus, comprising:
a captured image acquisition unit configured to acquire a captured image captured by an imaging device configured to image a subject;
a movement determination unit configured to determine magnitude of relative movement between the imaging device and the subject;
an image processing unit configured to execute image processing on the captured image to output a processed image;
an image generation unit configured to generate, based on the processed image, an image for diagnosis; and
an estimation unit configured to estimate a diagnostic candidate area serving as a diagnostic candidate in the processed image, by executing an estimation process for the processed image using a trained model, wherein
an input frame rate for the processed image with which the image generation unit sequentially executes a process of generating the image for diagnosis, and an input frame rate for the processed image sequentially input to the estimation unit differ from each other according to the magnitude of the movement.

2. The image diagnosis assistance apparatus according to claim 1, wherein the image generation unit is configured to generate the image for diagnosis by executing high-definition processing according to image quality of the processed image using the trained model.

3. The image diagnosis assistance apparatus according to claim 1, wherein the image generation unit is configured to generate, as the image for diagnosis, a high-definition image higher in definition than the processed image by executing high-definition processing on the processed image.

4. The image diagnosis assistance apparatus according to claim 1, further comprising a display control unit configured to generate a display image based on the image for diagnosis and the diagnostic candidate area.

5. The image diagnosis assistance apparatus according to claim 4, wherein the image generation unit is configured to generate, as the image for diagnosis, a high-definition image higher in image quality than the processed image by executing high-definition processing on the processed image.

6. The image diagnosis assistance apparatus according to claim 1, further comprising a frame selection unit configured to differ the input frame rate for the processed image with which the image generation unit sequentially executes the process of generating the image for diagnosis, and the input frame rate for the processed image sequentially input to the estimation unit from each other, according to the magnitude of the movement.

7. The image diagnosis assistance apparatus according to claim 1, further comprising an imaging control unit configured to change an imaging mode of the imaging device according to the magnitude of the movement.

8. The image diagnosis assistance apparatus according to claim 7, wherein the imaging control unit is configured to change a frame rate of imaging by the imaging device according to the magnitude of the movement.

9. The image diagnosis assistance apparatus according to claim 1, further comprising a light source control unit configured to change, according to the magnitude of the movement, an illumination mode of a light source device configured to supply illumination light to the subject.

10. The image diagnosis assistance apparatus according to claim 1, wherein when the magnitude of the movement is at a predetermined threshold or more, the input frame rate for the processed image sequentially input to the estimation unit is larger than the input frame rate for the processed image with which the image generation unit sequentially executes the process of generating the image for diagnosis.

11. The image diagnosis assistance apparatus according to claim 10, wherein when the magnitude of the movement is at the predetermined threshold or more, the image generation unit is configured to thin out frames of the processed image input and set the input frame rate for the processed image with which the image generation unit sequentially executes the process of generating the image for diagnosis to be smaller than the input frame rate for the processed image sequentially input to the estimation unit.

12. The image diagnosis assistance apparatus according to claim 1, further comprising:
a display control unit configured to generate a display image based on the image for diagnosis and the diagnostic candidate area, wherein
the display control unit is configured to, based on the image for diagnosis and the diagnostic candidate area that result from processing of the processed image of a same frame by the image generation unit and the estimation unit, generate the display image having the diagnostic candidate area superposed on the image for diagnosis.

13. The image diagnosis assistance apparatus according to claim 1, further comprising:
a display control unit configured to generate a display image based on the image for diagnosis and the diagnostic candidate area, wherein
the display control unit is configured to, based on the image for diagnosis and the diagnostic candidate area that result from processing of the processed image of a different frame by the image generation unit and the estimation unit, generate the display image including the image for diagnosis and information indicating a frame from which the diagnostic candidate area has been estimated.

14. The image diagnosis assistance apparatus according to claim 1, further comprising:
a display control unit configured to generate a display image based on the image for diagnosis and the diagnostic candidate area, wherein
the display control unit is configured to, based on the image for diagnosis and the diagnostic candidate area that result from processing of the processed image of a different frame by the image generation unit and the estimation unit, generate the display image having the diagnostic candidate area superposed on the image for diagnosis generated from the processed image of a frame nearest to the processed image of a frame from which the diagnostic candidate area has been estimated.

15. The image diagnosis assistance apparatus according to claim 1, wherein the movement determination unit is configured to determine the magnitude of the movement based on output of at least one of an acceleration sensor or an angular velocity sensor provided in the imaging device.

16. The image diagnosis assistance apparatus according to claim 1, wherein the movement determination unit is configured to determine the magnitude of the movement based on the processed image.

17. The image diagnosis assistance apparatus according to claim 1, wherein the estimation unit is configured to change the trained model according to the magnitude of the movement.

18. The image diagnosis assistance apparatus according to claim 1, wherein the estimation unit is configured to estimate, as the diagnostic candidate area, an area having reliability at a reliability threshold or more, among reliability of each predetermined area in the processed image output by the estimation process, and change the reliability threshold according to the magnitude of the movement.

19. The image diagnosis assistance apparatus according to claim 1, wherein the estimation unit is configured to estimate, as the diagnostic candidate area, an area where reliability is consecutively at a reliability threshold or more for a predetermined number of frames among reliability of each predetermined area in the processed image output by the estimation process, and change the predetermined number of frames according to the magnitude of the movement.

20. An image diagnosis assistance apparatus, comprising:
a captured image acquisition unit configured to acquire a captured image captured by an imaging device configured to image a subject;
a movement determination unit configured to determine magnitude of relative movement between the imaging device and the subject;
an image processing unit configured to execute image processing on the captured image to output a processed image;
an image generation unit configured to generate a high-definition image higher in definition than the processed image by executing high-definition processing on the processed image;
an estimation unit configured to estimate a diagnostic candidate area serving as a diagnostic candidate in the processed image by executing an estimation process for the processed image using a trained model; and
a display control unit configured to generate a display image based on the high-definition image and the diagnostic candidate area, wherein
the display control unit is configured to change a form of the display image according to the magnitude of the movement.

21. The image diagnosis assistance apparatus according to claim 20, wherein when the magnitude of the movement is at a predetermined threshold or more, an input frame rate for the processed image sequentially input to the estimation unit is larger than an input frame rate for the processed image on which the image generation unit sequentially executes the high-definition processing.

22. The image diagnosis assistance apparatus according to claim 21, wherein the display control unit is configured to:
when the magnitude of the movement is less than the predetermined threshold, based on the high-definition image and the diagnostic candidate area that result from processing of the processed image of a same frame by the image generation unit and the estimation unit, generate the display image having the diagnostic candidate area superposed on the high-definition image; and
when the magnitude of the movement is at the predetermined threshold or more, based on the high-definition image and the diagnostic candidate area that result from processing of the processed image of a same frame by the image generation unit and the estimation unit, generate the display image having the diagnostic candidate area superposed on the high-definition image, and based on the high-definition image and the diagnostic candidate area that result from processing of the processed image of a different frame by the image generation unit and the estimation unit, generate the display image including the high-definition image and information indicating a frame from which the diagnostic candidate area has been estimated.

23. An image diagnosis assistance system, comprising:
an imaging device configured to generate a captured image by imaging a subject; and
an image diagnosis assistance apparatus configured to process the captured image, wherein
the image diagnosis assistance apparatus comprises:
a captured image acquisition unit configured to acquire the captured image;
a movement determination unit configured to determine magnitude of relative movement between the imaging device and the subject;
an image processing unit configured to execute image processing on the captured image to output a processed image;
an image generation unit configured to generate a high-definition image higher in definition than the processed image by executing high-definition processing on the processed image; and
an estimation unit configured to estimate a diagnostic candidate area serving as a diagnostic candidate in the processed image by executing an estimation process for the processed image using a trained model, and
an input frame rate for the processed image on which the image generation unit sequentially executes the high-definition processing and an input frame rate for the processed image sequentially input to the estimation unit differ from each other according to the magnitude of the movement.

24. An image diagnosis assistance method executed by an image diagnosis assistance apparatus, the image diagnosis assistance method comprising:
a step of acquiring a captured image captured by an imaging device configured to image a subject;
a step of determining magnitude of relative movement between the imaging device and the subject;
a step of executing image processing on the captured image to output a processed image;
a step of generating a high-definition image higher in definition than the processed image by executing high-definition processing on the processed image; and
a step of estimating a diagnostic candidate area serving as a diagnostic candidate in the processed image by executing an estimation process for the processed image using a trained model, wherein
an input frame rate for the processed image on which the high-definition processing is sequentially executed at an image generation unit configured to execute the high-definition processing and an input frame rate for the processed image sequentially input to an estimation unit configured to execute the estimation process differ from each other according to the magnitude of the movement.
